# EUROPÄISCHE PATENTANMELDUNG

(11) **EP 3 020 329 A1**
(43) Veröffentlichungstag der Anmeldung: **18.05.2016**
(21) Anmeldenummer: 15194077.2
(22) Anmeldetag: 11.11.2015
(51) Int. Cl.: A61B 5/00, G06F 19/00

(54) **BILDGEBUNGSSYSTEM FÜR ANALYTISCHE ZWECKE**

(30) Priorität: 11.11.2014 DE 102014116442
(71) Anmelder: Unger, Rico, 80999 München (DE)
(72) Erfinder: Unger, Rico, 80999 München (DE)
(74) Vertreter: Kunz, Herbert

(57) **Zusammenfassung**

Die vorliegende Erfindung betrifft ein Bildgebungssystem für analytische Zwecke, das folgende Komponenten beinhaltet: ein mobiles Kamerasystem, einen lokalen Server, der drahtlos mit dem mobilen Kamerasystem verbunden ist, und wenigstens ein Peripheriegerät (Client). Die vorliegende Erfindung betrifft ferner die Verwendung der erfindungsgemäßen Bildgebungssysteme mit dem Schwerpunkt auf dem medizinischanalytischen Gebiet, insbesondere zur Ermittlung eines Zustandes menschlicher oder tierischer Haut, und ein Verfahren zur Ermittlung des Zustands menschlicher oder tierischer Haut.

## Beschreibung

Die vorliegende Erfindung betrifft ein Bildgebungssystem für analytische Zwecke, das folgende Komponenten beinhaltet: ein mobiles Kamerasystem bzw. eine mobile Kamera, einen lokalen Server, der drahtlos mit dem mobilen Kamerasystem verbunden ist und wenigstens ein Peripheriegerät (Client), das drahtlich oder drahtlos mit dem lokalen Server in Verbindung steht. Die vorliegende Erfindung betrifft ferner die Verwendung der erfindungsgemäßen Bildgebungssysteme mit dem Schwerpunkt auf dem medizinischanalytischen Gebiet, insbesondere zur Ermittlung eines Zustandes menschlicher oder tierischer Haut (Dermatoskopie). Ferner betrifft die vorliegende Erfindung ein Verfahren zur Ermittlung des Zustands menschlicher oder tierischer Haut.

Bildgebungssysteme - insbesondere Bildgebungssysteme im Bereich der medizinischen Diagnostik - werden in einem sehr breiten Anwendungsgebiet in modernen Einrichtungen des Gesundheitswesens eingesetzt. Solche Systeme stellen wertvolle Werkzeuge zur Identifikation, Diagnose und Behandlung eines Gesundheitszustands bereit. In vielen Fällen, wird die endgültige Diagnose erst dann gestellt und die Behandlung erst dann begonnen oder fortgesetzt, nachdem der behandelnde Arzt oder Radiologe konventionelle Untersuchungen mit detaillierten Bildern relevanter Bereiche und Gewebe durch eine oder mehrere Bildgebungsmodalitäten abgeschlossen hat.

Ein wichtiges Segment wird dabei durch Bildgebungssysteme für die Diagnostik der menschlichen oder tierischen Haut abgedeckt, da die korrekte visuelle Primärdiagnostik einer pigmentierten oder unpigmentierten präcancerösen oder cancerösen Hautläsionen einen entscheidenden Ausgangspunkt für eine erfolgreiche Behandlung darstellen. Wichtige Instrumente stellen dabei Dermatoskope bzw. Videodermatoskopie-Systeme dar.

Die Dermatoskopie bzw. Videodermatoskopie ist ein nichtinvasives Untersuchungsverfahren der Dermatologie, das insbesondere zur Früherkennung von bösartigen Tumoren der Haut (Melanomen) eingesetzt wird. Bei der vorbeugenden Untersuchung der Muttermale eines Patienten kann durch den Einsatz von Auflichtmikroskopen/Dermatoskopen - ein Präzisionsinstrument mit justierbarem Abstand, spezieller Kontaktplatte und Kontaktmedium - die Diagnostik pigmentierter und nichtpigmentierter Hauttumore grundlegend verbessert und dem Patienten unnötige operative Eingriffe oder Bestrahlungen erspart werden. Durch Kontaktmedien und den Einsatz von polarisiertem Licht, können zudem Details erfasst und daneben tiefere Hautschichten analysiert werden.

Eine wesentliche Weiterentwicklung der analogen Dermatoskope stellt die Videodermatoskopie dar. Während LED- (Licht emittierende Dioden) Beleuchtung, polarisiertes Licht und teilweise der Anschluss von Digitalkameras bei analogen Dermatoskopen zum Standard gehört, erweitert die digitale Videodermatoskopie die Untersuchung um die Bilddarstellung am Monitor, den Vergleich von Verlaufsbildern, die Archivierung aufgenommener Bilder in einer Datenbank, die Demonstration von Veränderungen vor dem Patienten und ggf. die Berechnung von bildanalytischen Kriterien. Die Videodermatoskopie, bei der das Bild einer kabelgebundenen Videokamera an einen angeschlossen Computermonitor wiedergegeben wird, ist inzwischen ein gut etabliertes Verfahren.

Die Videodermatoskopie basiert auf der klassischen analogen Dermatoskopie. Der Unterschied besteht darin, dass der Betrachter die zu untersuchende Stelle nicht durch die analoge "Lupe", sondern am Bildschirm des angeschlossenen Computers betrachtet. Dadurch wird eine Bildaufnahme, eine Vergrößerung und eine Archivierung und Verlaufskontrolle der untersuchten Läsionen ermöglicht. Die archivierten digitalen Bilder können von speziellen Vergleichs- und Analyseprogrammen weiter analysiert werden.

Die Bildauswertung basiert häufig auf der ABCDE-Regel (Asymmetrie, Begrenzung, Colour, Durchmesser und Erhabenheit bzw. Entwicklung). Problematisch ist dabei, dass die Wiedergabe von Farbtönen von verschiedenen Parametern wie z.B. der Beleuchtung, Aufnahmeart, digitaler Signalverarbeitung und von der Anzeige auf verschiedenen Monitoren abhängig ist. Aufnahmen mit polarisiertem und nicht-polarisiertem Licht oder Aufnahmen mit Immersionsflüssigkeit betonen unterschiedliche Bildmerkmale und erschweren zusätzlich eine Vergleichbarkeit der angefertigten Aufnahmen.

Die aus dem Stand der Technik bekannten Videodermatoskope weisen den schwerwiegenden Nachteil auf, dass sie groß sind und damit unflexibel hinsichtlich ihres jeweiligen Einsatzortes sind. Bei mobilen Systemen, die ebenfalls aus dem Stand der Technik bekannt sind, sind die einzelnen Komponenten wir z.B. Desktop-PC zumindest teilweise auf einem Gerätewagen verbaut, was allerdings ebenfalls mit einer Einschränkung der Mobilität verbunden ist. Bei allen Systemen ist die handgehaltene Kamera durch ein Kabel mit dem Personal Computer (PC) verbunden. Der Wechsel zwischen polarisiertem und nicht-polarisiertem Licht erfolgt durch den Wechsel von Kameraaufsätzen.

Die vorliegende Erfindung basiert somit auf der Aufgabe, ein Bildgebungssystem für analytische Zwecke - und insbesondere ein Videodermatoskopiesystem - zur Verfügung zu stellen, das die oben aufgezeigten Nachteile der aus dem Stand der Technik bereits bekannten Systeme wenigstens teilweise überwindet.

Die Aufgabe wird durch ein Bildgebungssystem für analytische Zwecke und insbesondere durch ein Videodermatoskopiesystem mit den Merkmalen des Anspruchs 1 gelöst. Bevorzugte Ausführungsformen werden durch die von Anspruch 1 abhängigen Unteransprüche wiedergegeben. Die nebengeordneten Patentansprüche 23 und 25 betreffen, die Verwendung des erfindungsgemäßen Bildgebungssystems und ein Verfahren zur Ermittlung eines Zustands menschlicher oder tierischer Haut.

Das erfindungsgemäße Bildgebungssystem verkörpert ein Videosystem bzw. ein Videodermatoskopiesystem zur Aufnahme, Archivierung und Verlaufskontrolle von Bildern - insbesondere von dermatoskopischen oder klinischen Bildern.

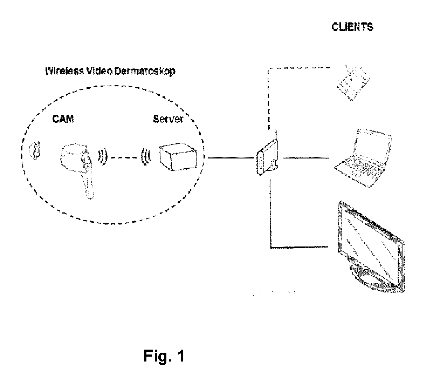

Ein Bildgebungssystem gemäß der vorliegenden Erfindung ist in der Fig. 1 wiedergegeben. Fig. 1 zeigt eine mobile Videokamera (Cam) - bzw. ein handgehaltenes Videodermatoskop - die/das über ein drahtloses Netzwerk (WLAN, Wireless Local Area Network) auf WPA-Basis (Wi-Fi Protected Access) mit dem lokalen Server verbunden ist. Die Serverfunktion kann von jedem handelsüblichen PC, der z.B. über einen Intel^{®}-Prozessor verfügt und der beispielsweise auf ein Linux^{®}-Betriebssystem oder ein anderes gängiges Betriebssystem (z.B. Windows^{®} oder OSx^{®}) zugreifen kann, übernommen werden.

Die dazugehörige Software ist auf dem lokalen Server installiert, und wird von - im Praxisnetzwerk vorhandenen - Clients (Peripheriegeräten - in Fig. 1 rechts dargestellt) unter Verwendung eines Web-Browsers bedient. In Fig. 1 ist die Verbindung mit den Clients in Form einer Funkverbindung vorgesehen - sie kann aber auch durch eine drahtliche Verbindung dargestellt werden. Die Clients sind betriebssystemunabhängig und können durch handelsübliche Personal Computer, Notebooks oder Tablets, die allesamt aus dem Stand der Technik in großer Zahl bekannt sind - verkörpert werden.

Die Kamera bzw. das Kamerasystem kann u.a. über folgende wesentliche Komponenten verfügen, wozu beispielsweise sog. CCD (Charge-Coupled Device) Sensor oder bevorzugt ein CMOS (Complementary Metal-Oxide-Semiconductor) Bildsensor zählt, der z.B. eine Auflösung von 5 MPixel besitzt. Daneben kann die Kamera zum Beispiel über einen integrierten Schaltkreis (Central Processing Unit, CPU) verfügen, der als eine Kombination aus einem sog. FPGA (Field Programmable Gate Array) oder DSP (Digital Signal Processor) und einem Mikrokontroller ausgebildet ist und der zur weiteren Verarbeitung der aufgenommenen Bilder mittels einer speziell angepassten Image-Pipeline (Debayering, Colour Correction, Gamma Korrektur, Bildkompression etc.) sowie zur Ansteuerung der Peripherie, Datenübertragung per WIFI und Umsetzung des User-Interface dient. Durch die Kombination der beschriebenen Beleuchtung mit der erfindungsgemäßen Image-Pipeline können die Bilder so konfiguriert bzw. korrigiert werden, dass ein optimales Bild entsteht, wodurch die zuvor genannten Probleme in ihren Auswirkungen abgemildert werden. Im Ergebnis wird damit die Vergleichbarkeit der mit dem erfindungsgemäßen System angefertigten Bilder sichergestellt.

Ferner verfügt die Kamera über ein WIFI Modul, welches z.B. wenigstens auf dem IEEE 802.11-Standard basiert, sowie beispielsweise über ein OLED- (Organic Light Emitting Diode) oder TFT- (Thin-Film Transistor-Display) Display zur Darstellung des Kamerabildes und des User-Interfaces. Daneben kann die Kamera über kapazitive Bedienelemente zur Steuerung der Kamerafunktionen (Start/Stopp, umschalten zwischen den Beleuchtungsmodi polarisiert, nicht-polarisiert und fusionierten) verfügen sowie beispielsweise über eine Qi-kompatible Ladeelektronik zum kontaktlosen Laden der integrierten Batterie in der Ladestation. Alle genannten Komponenten sind für sich allein aus dem Stand der Technik bekannt und können ggf. durch andere - äquivalente - Bauteile leicht ausgetauscht werden.

Die Optik der Kamera kann beispielsweise eine LED Beleuchtung, vorzugsweise bestehend aus z.B. vier neutralweißen LEDs, die ringförmig angeordnet und zueinander abgewinkelt sind, aufweisen. Zwei der vier LEDs sind mit einem Polarisationsfilter ausgestattet und ermöglichen so das elektronische Umschalten zwischen zwei LEDs mit polarisiertem Licht und zwei LEDs mit nicht-polarisiertem Licht. Die abgewinkelt angeordneten LEDs ermöglichen ein homogen ausgeleuchtetes Objektfeld, das frei von Reflexionen ist.

Ferner umfasst die Optik eine mikro- und makroskopische Optik mit integriertem Polfilter, welche die Aufnahme von mikroskopischen und/oder dermatologischen Bildern sowie von makroskopischen Übersichtsaufnahmen großflächiger Merkmale ermöglicht.

Auf der mechanischen Seite verfügt die Kamera u.a. beispielsweise über einen auswechselbaren, konusförmigen oder pyramidenstumpfartigen Kontaktkopf mit einer integrierten Kontaktscheibe zur Auflage auf die Haut des Patienten. Zum leichteren Auswechseln kann der Kontaktkopf beispielsweise mit einem Magnetanschluss verfügen.

Die Applikationssoftware wird zweckmäßigerweise auf dem lokalen Server hinterlegt und sollte zweckmäßigerweise über einen geeigneten WEB-Browser von jedem beliebigen Client erreichbar bzw. bedienbar sein.

Die Software soll unter einem allgemeinen Aspekt dem Dermatologen einen optimierten und seinen Arbeitsablauf unterstützenden Workflow ermöglichen, wozu u.a. das Vorhandensein bestimmter Funktionen gehört- z.B.:
- Verschiedene Modi -wie beispielsweise , Screening, Baseline (Erstaufnahme) und Follow-Up.
- Verzögerungsfreies Darstellen von Life-Bilddaten der Kamera im Browser und Vergleichen in der Vergangenheit angefertigten Aufnahmen.
- Von relevanten Merkmalen können hochauflösende Aufnahmen anhand einer Bodymap einer bestimmten Körperstelle zugeordnet, mit Kommentar versehen und in einer Datenbank gespeichert werden.
- Bei einer Follow-Up Untersuchung wird der Arzt automatisch zu den bei der Erstuntersuchung aufgenommenen Merkmalen geführt, kann diese vergleichen und weitere Aufnahmen hinzufügen.
- Einzelne Untersuchungen oder ganze Datensätze können in Form von Reports oder als Backup exportiert werden.
- Patientendaten werden verwaltet; eine Anbindung an ein Bezahlsystem ist integriert.

Somit betrifft die vorliegende Erfindung ein Bildgebungssystem für analytische Zwecke umfassend folgende Bestandteile
a) ein mobiles Kamerasystem,
b) einen lokalen Server, der drahtlos mit dem mobilen Kamerasystem verbunden ist,
c) wenigstens einen Client (Peripheriegerät), der drahtlich oder drahtlos mit dem lokalen Server verbunden ist.

Bevorzugt betrifft die vorliegende Erfindung das oben beschriebene Bildgebungssystem, worin der Client durch einen Personal Computer (PC), ein Notebook oder durch ein Tablet verkörpert werden kann.

Weiterhin betrifft die vorliegende Erfindung bevorzugt eines der oben beschriebenen Bildgebungssysteme, worin die drahtlose Verbindung zwischen dem mobilen Kamerasystem und dem lokalen Server über ein drahtloses Netzwerk (WLAN, Wireless Local Area Network) auf WPA-Basis (Wi-Fi Protected Access) erfolgt.

Weiterhin betrifft die vorliegende Erfindung bevorzugt eines der oben beschriebenen Bildgebungssysteme, worin die drahtlose Verbindung zwischen dem Server und wenigstens einem Client über ein drahtloses Netzwerk (WLAN, Wireless Local Area Network) gemäß WPA-Standard (Wi-Fi Protected Access) erfolgt.

Weiterhin betrifft die vorliegende Erfindung bevorzugt eines der oben beschriebenen Bildgebungssysteme, worin das mobile Kamerasystem einen CMOS-Bildsensor (Complementary Metal-Oxide-Semiconductor-Bildsensor) aufweist.

Weiterhin betrifft die vorliegende Erfindung bevorzugt eines der oben beschriebenen Bildgebungssysteme, worin das mobile Kamerasystem einen integrierten Schaltkreis (Central Processing Unit, CPU) aufweist, der eine Kombination aus einem FPGA (Field Programmable Gate Array) oder DSP (Digital Signal Processor) und einem Mikrokontroller umfasst.

Weiterhin betrifft die vorliegende Erfindung bevorzugt eines der oben beschriebenen Bildgebungssysteme, worin das mobile Kamerasystem ein Wi-Fi-Modul gemäß dem IEEE 802.11-Standard aufweist.

Weiterhin betrifft die vorliegende Erfindung bevorzugt eines der oben beschriebenen Bildgebungssysteme, worin das mobile Kamerasystem ein TFT-Display (Thin-Film Transistor-Display) aufweist.

Weiterhin betrifft die vorliegende Erfindung bevorzugt eines der oben beschriebenen Bildgebungssysteme, worin das mobile Kamerasystem über eine Qi-kompatible Ladeelektronik (kabellose Energieübertragung) verfügt.

Weiterhin betrifft die vorliegende Erfindung bevorzugt eines der oben beschriebenen Bildgebungssysteme, worin das Bildgebungssystem ein Videodermaskopiesystem darstellt und das mobile Kamerasystem ein Videodermatoskop ist.

Weiterhin betrifft die vorliegende Erfindung bevorzugt das oben genannte Videodermaskopiesystem, worin das Videodermatoskop Beleuchtungsmittel zur Beleuchtung der Haut aufweist.

Weiterhin betrifft die vorliegende Erfindung bevorzugt eines der oben beschriebenen Videodermaskopiesysteme, worin die Beleuchtungsmittel durch eine ringförmige, eine konzentrische Ringbeleuchtung oder durch eine Beleuchtung am Umfang des optischen Systems bzw. eines nicht-planaren Polfilters in Form eines in das Gehäuse eingelassenen Fensters zur gleichmäßigen Ausleuchtung des zu vermessenden Hautbereichs verkörpert werden.

Weiterhin betrifft die vorliegende Erfindung bevorzugt eines der oben beschriebenen Videodermaskopiesysteme, worin die Beleuchtungsmittel durch LEDs (Licht emittierende Dioden) verkörpert werden.

Weiterhin betrifft die vorliegende Erfindung bevorzugt eines der oben beschriebenen Videodermaskopiesysteme, gemäß dem das Licht wenigstens eines Beleuchtungsmittels, insbesondere einer LED, durch mindestens ein erstes polarisierendes Element, insbesondere durch einen Polarisationsfilter, das bzw. der in Form einer Kunststoff- oder Glasscheibe, eines Films oder in Form einer Kunststoffplatte ausgebildet sein kann, geleitet wird, so dass das auf die Hautoberfläche einwirkende Licht eine definierte Schwingungsebene aufweist.

Weiterhin betrifft die vorliegende Erfindung bevorzugt eines der oben beschriebenen Videodermaskopiesysteme, worin das Videodermatoskop über wenigstens zwei LEDs mit einem Polarisationsfilter und über wenigstens zwei LEDs ohne Polarisationsfilter verfügt.

Weiterhin betrifft die vorliegende Erfindung bevorzugt eines der oben beschriebenen Videodermaskopiesysteme, worin das Videodermatoskop über eine mikroskopische- und makrospkopische Optik mit einem integrierten Polfilter verfügt, der in den Strahlengang integriert ist.

Weiterhin betrifft die vorliegende Erfindung bevorzugt eines der oben beschriebenen Videodermaskopiesysteme, worin das Videodermatoskop einen auswechselbaren Kontaktkopf mit integrierter Kontaktscheibe für die Auflage auf die menschliche oder tierische Haut aufweist.

Weiterhin betrifft die vorliegende Erfindung bevorzugt eines der oben beschriebenen Videodermaskopiesysteme, worin der Kontaktkopf konusförmig ist oder die Form eines Pyramidenstumpfes hat.

Weiterhin betrifft die vorliegende Erfindung bevorzugt eines der oben beschriebenen Videodermaskopiesysteme, worin das Videodermatoskop mindestens über eine drahtlose Schnittstelle zur Datenübertragung verfügt.

Weiterhin betrifft die vorliegende Erfindung bevorzugt eines der oben beschriebenen Videodermaskopiesysteme, das wenigstens ein Mittel zur sequentiellen oder gleichzeitigen Auswertung und Übertragung mehrerer nacheinander oder miteinander erfasster Bildinformationen, insbesondere Bilder mit polarisiertem Licht, Elevationsdaten in Echtzeit oder zu einem späteren Zeitpunkt aufweist.

Weiterhin betrifft die vorliegende Erfindung bevorzugt eines der oben beschriebenen Videodermaskopiesysteme, das wenigstens ein Auswertungsmittel zur automatischen Auswertung der erfassten Daten aufweist.

Weiterhin betrifft die vorliegende Erfindung bevorzugt eines der oben beschriebenen Videodermaskopiesysteme, worin das Auswertungsmittel auf der ABCD(E)-Regel (Asymmetrie, Begrenzung, Colour (Farbe), Durchmesser und ggf. Erhabenheit bzw. Entwicklung) basiert.

Gemäß einem zweiten Aspekt betrifft die vorliegende Erfindung die Verwendung des erfindungsgemäßen Videodermatoskopiesystems zur Ermittlung eines Zustands menschlicher oder tierischer Haut.

Daneben betrifft die vorliegende Erfindung gemäß dem obigen zweiten Aspekt vorzugsweise die Verwendung des erfindungsgemäßen Videodermatoskopiesystems für die Ermittlung eines Zustands menschlicher oder tierischer Haut, welche die Bestimmung der Asymmetrie, der Abmessungen (Begrenzung), der Farbe oder des Durchmessers einer Pigmentierung oder der Erhabenheit bzw. der Entwicklung der menschlichen oder tierischen Haut beinhaltet.

Gemäß einem dritten Aspekt betrifft die vorliegende Erfindung ein Verfahren zur Ermittlung eines Zustands menschlicher oder tierischer Haut unter Verwendung des erfindungsgemäßen Videodermatoskopiesystems, welches die folgenden Schritte umfasst:
a) Bestimmung der Asymmetrie, der Abmessungen (Begrenzung), der Farbe oder des Durchmessers einer Pigmentierung oder der Erhabenheit bzw. der Entwicklung der menschlichen oder tierischen Haut mit einem Videodermatoskop;
b) drahtlose Übermittlung der in Schritt a) der erfassten Daten an einen lokalen Server;
c) Auswertung der erfassten Daten mit wenigstens einem Auswertungsmittel;
d) Darstellung der Auswertung der erfassten Daten, vorzugsweise mittels eines Monitors und ggf.
e) Speicherung bzw. Archivierung der erfassten bzw. ausgewerteten Daten in einem Speichermedium, vorzugsweise in dem Server oder in einem Client (Peripheriegerät) und
f) ggf. Übermittlung der Daten an einen Client.

Des Weiteren betrifft die vorliegende Erfindung vorzugsweise das oben beschriebene Verfahren, in dem die Bestimmung bzw. Nachverfolgung der Asymmetrie, der Abmessungen (Begrenzung), der Farbe oder des Durchmessers einer Pigmentierung oder der Erhabenheit bzw. der Entwicklung der menschlichen oder tierischen Haut mittels in Teilschritt a) ohne den Wechsel von Kameraaufsätzen mittels wenigstens zwei LEDs mit einem Polarisationsfilter und mittels wenigstens zwei LEDs ohne Polarisationsfilter erfolgt. und einem Polarisationsfilter, der das aufzunehmende Licht polarisiert bzw. filtriert, erfolgt.

Des Weiteren betrifft die vorliegende Erfindung vorzugsweise eines der oben beschriebenen Verfahren, in dem die Steuerung der Funktionen des Videodermatoskops mittels kapazitiver Bedienelemente erfolgt.

Des Weiteren betrifft die vorliegende Erfindung vorzugsweise eines der oben beschriebenen Verfahren, in dem die Übermittlung der erfassten Daten in Teilschritt b) in Form von Bildern mittels einer angepassten Image-Pipeline, bevorzugt umfassend die Schritte Debayering, Colour Correction, Gamma Korrektur, Bildkompression, erfolgt.

Des Weiteren betrifft die vorliegende Erfindung vorzugsweise eines der oben beschriebenen Verfahren, in dem die drahtlose Übermittlung in Teilschritt b) innerhalb eines Netzwerks (WLANs) auf der Basis eines WiFi-Standards erfolgt.

Des Weiteren betrifft die vorliegende Erfindung vorzugsweise eines der oben beschriebenen Verfahren, in dem die Auswertung gemäß Teilschritt c) auf der Basis der ABCD(E)-Regel (Asymmetrie, Begrenzung, Colour (Farbe), Durchmesserund ggf. Erhabenheit bzw. Entwicklung) erfolgt.

Des Weiteren betrifft die vorliegende Erfindung vorzugsweise eines der oben beschriebenen Verfahren, in welchem die Übertragung zum Client über den Webbrowser erfolgt.

## Patentansprüche

1. Bildgebungssystem für analytische Zwecke umfassend folgende Bestandteile
a) ein mobiles Kamerasystem,
b) einen lokalen Server, der drahtlos mit dem mobilen Kamerasystem verbunden ist,
c) wenigstens einen Client (Peripheriegerät), der drahtlich oder drahtlos mit dem lokalen Server verbunden ist.

2. Bildgebungssystem, gemäß Anspruch 1, **dadurch gekennzeichnet, dass** der Client durch einen Personal Computer (PC), ein Notebook oder durch ein Tablet verkörpert werden kann und / oder vorzugsweise
die drahtlose Verbindung zwischen der dem mobilen Kamerasystem und dem lokalen Server über ein drahtloses Netzwerk (WLAN, Wireless Local Area Network) auf WPA-Basis (Wi-Fi Protected Access) erfolgt und / oder vorzugsweise
die drahtlose Verbindung zwischen dem Server und wenigstens einem Client über ein drahtloses Netzwerk (WLAN, Wireless Local Area Network) gemäß WPA-Standard (Wi-Fi Protected Access) erfolgt.

3. Bildgebungssystem, gemäß einem der Ansprüche 1 oder 2, **dadurch gekennzeichnet, dass** das mobile Kamerasystem einen CMOS-Bildsensor (Complementary Metal-Oxide-Semiconductor-Bildsensor) aufweist, und / oder vorzugsweise
das mobile Kamerasystem einen integrierten Schaltkreis (Central Processing Unit, CPU) aufweist, der eine Kombination aus einem FPGA (Field Programmable Gate Array) oder DSP (Digital Signal Processor) und einem Mikrokontroller umfasst und / oder vorzugsweise
das mobile Kamerasystem ein Wi-Fi-Modul gemäß dem IEEE 802.11-Standard aufweist und / oder vorzugsweise
das mobile Kamerasystem ein TFT-Display (Thin-Film Transistor-Display) aufweist und / oder vorzugsweise
das mobile Kamerasystem über eine Qi-kompatible Ladeelektronik (kabellose Energieübertragung) verfügt und / oder vorzugsweise
das Bildgebungssystem ein Videodermaskopie-System darstellt und das mobile Kamerasystem ein Videodermatoskop ist und / oder vorzugsweise
das Videodermatoskop Beleuchtungsmittel zur Beleuchtung der Haut aufweist und / oder vorzugsweise
die Beleuchtungsmittel durch eine ringförmige, eine konzentrische Ringbeleuchtung oder durch eine Beleuchtung am Umfang des optischen Systems bzw. eines nicht-planaren Polfilters in Form eines in das Gehäuse eingelassenen Fensters zur gleichmäßigen Ausleuchtung des zu vermessenden Hautbereichs verkörpert werden und / oder vorzugsweise
die Beleuchtungsmittel durch LEDs (Licht emittierende Dioden) verkörpert werden und / oder vorzugsweise
das Licht wenigstens eines Beleuchtungsmittels, insbesondere einer LED, durch mindestens ein erstes polarisierendes Element, insbesondere durch einen Polarisationsfilter, das bzw. der in Form einer Kunststoff- oder Glasscheibe, eines Films oder in Form einer Kunststoffplatte ausgebildet sein kann, geleitet wird, so dass das auf die Hautoberfläche einwirkende Licht eine definierte Schwingungsebene aufweist und / oder vorzugsweise
das Videodermatoskop über wenigstens zwei LEDs mit einem Polarisationsfilter und über wenigstens zwei LEDs ohne Polarisationsfilter verfügt und / oder vorzugsweise das Videodermatoskop über eine mikroskopische und makrospkopische Optik mit einem integrierten Polfilter verfügt, der in den Strahlengang integriert ist und / oder vorzugsweise
das Videodermatoskop einen auswechselbaren Kontaktkopf mit integrierter Kontaktscheibe für die Auflage auf die Haut aufweist und / oder vorzugsweise
der Kontaktkopf konusförmig ist oder die Form eines Pyramidenstumpfes hat und / oder vorzugsweise
das Videodermatoskop mindestens über eine drahtlose Schnittstelle zur Datenübertragung verfügt und / oder vorzugsweise
es wenigstens ein Mittel zur sequenziellen oder gleichzeitigen Auswertung und Übertragung mehrerer nacheinander oder miteinander erfasster Bildinformationen, insbesondere Bilder mit polarisiertem Licht, Elevationsdaten, in Echtzeit oder zu einem späteren Zeitpunkt aufweist.

4. Bildgebungssystem, gemäß einem der Ansprüche 2 oder 3, **dadurch gekennzeichnet, dass** es wenigstens ein Auswertungsmittel zur automatischen Auswertung der erfassten Daten aufweist.

5. Bildgebungssystem, gemäß Anspruch 4, **dadurch gekennzeichnet, dass** das Auswertungsmittel auf der ABCD(E)-Regel (Asymmetrie, Begrenzung, Colour (Farbe), Durchmesser und ggf. Erhabenheit bzw. Entwicklung) basiert.

6. Verwendung eines Bildgebungssystems gemäß einem der Ansprüche 1 bis 5 zur Ermittlung eines Zustands menschlicher oder tierischer Haut.

7. Verwendung eines Bildgebungssystems gemäß Anspruch 6, **dadurch gekennzeichnet, dass** die Ermittlung des Zustands menschlicher oder tierischer Haut die Bestimmung der Asymmetrie, der Abmessungen (Begrenzung), der Farbe oder des Durchmessers einer Pigmentierung oder der Erhabenheit bzw. Entwicklung der menschlichen oder tierischen Haut beinhaltet.

8. Verfahren zur Ermittlung des Zustands menschlicher oder tierischer Haut unter Verwendung des Videodermatoskopie-Systems gemäß einem der Ansprüche 1 bis 5 umfassend die folgenden Schritte:
a) Bestimmung der Asymmetrie, der Abmessungen (Begrenzung), der Farbe oder des Durchmessers einer Pigmentierung oder der Erhabenheit der menschlichen oder tierischen Haut mit einem Videodermatoskop,
b) drahtlose Übermittlung der in Schritt a) der erfassten Daten an einen lokalen Server,
c) Auswertung der erfassten Daten mit wenigstens einem Auswertungsmittel,
d) Darstellung der Auswertung der erfassten Daten, vorzugsweise mittels eines Monitors und ggf.
e) Speicherung bzw. Archivierung der erfassten bzw. ausgewerteten Daten in einem Speichermedium, vorzugsweise in dem Server oder in einem Client (Peripheriegerät); und
f) ggf. Übermittlung der Daten an einen Client

9. Verfahren gemäß Anspruch 8, **dadurch gekennzeichnet, dass** Bestimmung bzw. Nachverfolgung der Asymmetrie, der Abmessungen (Begrenzung), der Farbe oder des Durchmessers einer Pigmentierung oder der Erhabenheit bzw. der Entwicklung der menschlichen oder tierischen Hautmittels in Teilschritt a) ohne den Wechsel von Kameraaufsätzen mittels wenigstens zweier LEDs mit einem Polarisationsfilter und mittels wenigstens zweier LEDs ohne Polarisationsfilter und einem Polarisationsfilter, der das aufzunehmende Licht polarisiert bzw. filtriert, erfolgt und / oder vorzugsweise
die Steuerung der Funktionen des Videodermatoskops mittels kapazitiver Bedienelemente erfolgt und / oder vorzugsweise
die Übermittlung der erfassten Daten in Teilschritt b) in Form von Bildern mittels einer angepassten Image-Pipeline, vorzugsweise umfassend die Schritte Debayering, Colour Correction, Gamma Korrektur, Bildkompression, erfolgt.

10. Verfahren gemäß einem der Ansprüche 8 oder 9, **dadurch gekennzeichnet, dass** die drahtlose Übermittlung in Teilschritt b) innerhalb eines Netzwerks (WLANs) auf der Basis eines Wi-Fi-Standards erfolgt.

11. Verfahren, gemäß einem der Ansprüche 8 bis 10, **dadurch gekennzeichnet, dass** die Auswertung gemäß Teilschritt c) auf der Basis der ABCD(E)-Regel (Asymmetrie, Begrenzung, Colour (Farbe), Durchmesserund und ggf. Erhabenheit bzw. Entwicklung) erfolgt.

12. Verfahren nach einem der Ansprüche 8 bis 11, **dadurch gekennzeichnet, dass** die Übertragung zum Client über einen Webbrowser erfolgt.
